# EUROPEAN PATENT APPLICATION

(11) **EP 4 670 621 A1**
(43) Date of publication of application: **31.12.2025**
(21) Application number: 24785188.4
(22) Date of filing: 03.04.2024
(51) Int. Cl.: A61B 5/00, A61B 5/346, G16H 50/20

(54) **ARTIFICIAL INTELLIGENCE-BASED DISEASE PREDICTION METHOD, PROGRAM, AND DEVICE**

(30) Priority: 03.04.2023 KR 20230043528
(71) Applicant: Medical AI Co., Ltd., Seoul 06180 (KR)
(72) Inventor: KWON, Joonmyoung, Seoul 06180 (KR)
(74) Representative: Patentanwaltskanzlei Matschnig & Forsthuber OG
(86) International application number: PCT/KR2024/004280
(87) International publication number: WO 2024/210483

(57) **Abstract**

Provided, according to one embodiment of the present disclosure, are an artificial intelligence-based disease prediction method performed by a computing device, a program, and a device. The method may comprise: obtaining a biosignal of a subject to be predicted; generating physiological information of the subject on the basis of the obtained biosignal using a pre-trained first neural network model; and generating prediction information regarding disease occurrence in the subject on the basis of the obtained biosignal and the generated physiological information using a pre-trained second neural network model.

## Description

### TECHNICAL FIELD

The present disclosure relates to artificial intelligence technology in the medical field, and more particularly, to an artificial intelligence-based disease diagnosis method capable of achieving high-accuracy analysis even in medical data areas where sufficient labeled data are not available.

### BACKGROUND ART

When analyzing biosignals with artificial intelligence technology, it may improve the accuracy of the analysis to utilize various physiological information in addition to the biosignals. For example, when analyzing for the presence of myocardial infarction, the accuracy tends to be higher when additional information, namely physiological information such as age, sex, and weight, is used together with an electrocardiogram signal as input values, than when the electrocardiogram signal alone is used as an input value.

A large amount of data is required for training an artificial intelligence model. Therefore, as described above, in order to utilize various additional information together with biosignals for analysis, sufficient data in which additional information is matched with biosignals is required in order to train an artificial intelligence model. However, in an actual medical environment, the amount of labeled data in which additional information is matched with biosignals is very limited. For example, the amount of data in which additional information, such as age, sex, and weight, is all recorded together with an electrocardiogram signal for myocardial infarction may be significantly smaller than the number of electrocardiogram signals for myocardial infarction. Furthermore, when an artificial intelligence model is used in the actual field, although to provide an immediate result after measuring an electrocardiogram signal is desirable, requiring the user to enter additional information may impose an extra burden.

### DETAILED DESCRIPTION OF THE INVENTION

### Technical Problem

The present disclosure provides an artificial intelligence-based disease diagnosis method capable of achieving high-accuracy analysis even in medical data areas where sufficient labeled data are not available.

However, the technical problem which the present disclosure intends to address is not limited to the problem mentioned above, and other problems not mentioned herein may be clearly understood on the basis of the following description.

### Technical Solution

Provided, according to one embodiment of the present disclosure for addressing the above-described problem, is an artificial intelligence-based disease prediction method performed by a computing device. The method may comprise: obtaining a biosignal of a subject to be predicted; generating physiological information of the subject on the basis of the obtained biosignal using a pre-trained first neural network model; and generating prediction information regarding disease occurrence in the subject on the basis of the obtained biosignal and the generated physiological information using a pre-trained second neural network model.

Alternatively, the first neural network model may comprise a plurality of sub-models separately constructed for respective types of the physiological information.

Alternatively, the first neural network model may comprises: a first sub-model configured to receive the obtained biosignal and generate first additional information included in the physiological information; and a second sub-model configured to receive the obtained biosignal together with the generated first additional information, and generate second additional information included in the physiological information.

Alternatively, the first additional information may comprise at least one of age or sex of the subject. The second additional information may comprise at least one of blood pressure, blood analysis results, weight, height, or underlying disease of the subject.

Alternatively, the second neural network model may receive the obtained biosignal, the generated first additional information, and the generated second additional information, and calculate a probability of disease occurrence in the subject.

Alternatively, the step of generating prediction information regarding disease occurrence in the subject on the basis of the obtained biosignal and the generated physiological information using the pre-trained second neural network model may comprise: determining weights to be assigned to the plurality of additional information included in the generated physiological information according to a type of disease determined by a user input; and generating prediction information regarding occurrence of the disease determined by the user input, on the basis of the obtained biosignal and the plurality of additional information to which the determined weights are assigned, using the second neural network model.

Alternatively, the weights to be assigned to the plurality of additional information may vary depending on the type of disease, and may be dynamically selected from a weight database constructed based on analysis results of clinical experts according to the user input.

Alternatively, the second neural network model may comprise a plurality of sub-models separately constructed for respective types of diseases. When the type of disease is determined by the user input, a sub-model of the second neural network model corresponding to the determined type of disease may be activated.

Provided, according to one embodiment of the present disclosure for addressing the above-described problem, is a computer program stored in a computer-readable storage medium. The computer program, when executed by one or more processors, performs operations for predicting a disease based on artificial intelligence. The operations may comprise: obtaining a biosignal of a subject to be predicted; generating physiological information of the subject on the basis of the obtained biosignal using a pre-trained first neural network model; and generating prediction information regarding disease occurrence in the subject on the basis of the obtained biosignal and the generated physiological information using a pre-trained second neural network model.

Provided, according to one embodiment of the present disclosure for addressing the above-described problem, is a computing device for predicting a disease based on artificial intelligence. The computing device may comprise: a processor including at least one core; a memory including program codes executable by the processor; and a network unit configured to obtain a biosignal of a subject to be predicted. The processor is configured to generate physiological information of the subject on the basis of the obtained biosignal using a pre-trained first neural network model and to generate prediction information regarding disease occurrence in the subject on the basis of the obtained biosignal and the generated physiological information using a pre-trained second neural network model.

### Advantageous Effects

According to the present disclosure, it is possible to construct an artificial intelligence model capable of accurately predicting a disease of a subject to be analysed even though labeled data in which physiological information is matched with biosignals are not available. Furthermore, even if a user does not additionally input physiological information, an analysis based only on biosignals may be performed with an accuracy comparable to that achieved when both biosignals and physiological information are utilized.

### Brief Description of the Drawings

FIG. 1 is a block diagram illustrating a computing device according to one embodiment of the present disclosure.
FIG. 2 is a block diagram illustrating an operation process of a neural network model according to one embodiment of the present disclosure.
FIG. 3 is a block diagram illustrating a structure of a first neural network model according to one embodiment of the present disclosure.
FIG. 4 is a block diagram illustrating a structure of a first neural network model according to an alternative embodiment of the present disclosure.
FIG. 5 is a flowchart illustrating a process of generating prediction information according to one embodiment of the present disclosure.
FIG. 6 is a flowchart illustrating an artificial intelligence-based disease prediction method according to one embodiment of the present disclosure.

### Detailed Description of the Embodiments

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the description of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that has problem-solving capability through training. In this case, the neural network may have problem-solving capability by optimizing parameters connecting nodes or neurons through training. The neural network "model" may comprise a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, they are not used in the sense of limiting the content or the technical spirit of the present disclosure.

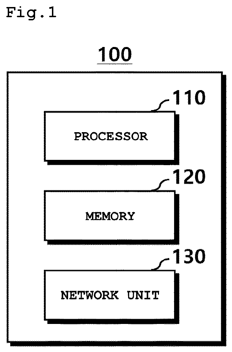

FIG. 1 is a block diagram illustrating a computing device according to one embodiment of the present disclosure.

The computing device 100 according to one embodiment of the present disclosure may be a hardware device or a part of a hardware device that performs the comprehensive processing and operation of data, or may be a software-based computing environment operating over a communication network. For example, the computing device 100 may be a server that is a main entity performing an intensive data processing function and sharing resources, or may be a client that shares resources through interaction with a server. Furthermore, the computing device 100 may be a cloud system in which pluralities of servers and clients comprehensively process data while interacting with each other. Since the above-described description is only one example related to the type of the computing device 100, the type of the computing device 100 may be configured in various manners within a range understandable to those skilled in the art based on the content of the present disclosure.

Referring to FIG. 1, the computing device 100 according to one embodiment of the present disclosure may comprise a processor 110, memory 120, and a network unit 130. However, since FIG. 1 shows only an example, the computing device 100 may comprise other components for implementing a computing environment. Furthermore, only some of the components disclosed above may be comprised in the computing device 100.

The processor 110 according to one embodiment of the present disclosure may be understood as a constituent unit comprising hardware and/or software for performing computing operation. For example, the processor 110 may read a computer program and perform data processing for machine learning. The processor 110 may process operation procedures such as processing input data for machine learning, extracting features for machine learning, and calculating errors based on backpropagation. The processor 110 for performing such data processing may comprise a central processing unit(CPU), a general purpose graphics processing unit(GPGPU), a tensor processing unit(TPU), an application specific integrated circuit(ASIC), or a field programmable gate array(FPGA). Since the types of the processor 110 described above are only examples, the type of the processor 110 may be configured in various manners within a range understandable to those skilled in the art based on the content of the present disclosure.

The memory 120 according to one embodiment of the present disclosure may be understood as a constituent unit comprising hardware and/or software for storing and managing data that is processed in the computing device 100. That is, the memory 120 may store any type of data generated or determined by the processor 110, and any type of data received by the network unit 130. For example, the memory 120 may comprise a storage medium of at least one type of a flash memory type, a hard disk type, a multimedia card micro type, a card type memory, a random access memory (RAM), a static random access memory (SRAM), a read-only memory(ROM), an electrically erasable programmable read-only memory(EEPROM), a programmable read-only memory(PROM), a magnetic memory, a magnetic disk, and an optical disk. Furthermore, the memory 120 may comprise a database system that controls and manages data in a predetermined system. Since the types of memory 120 described above are only examples, the type of the memory 120 may be configured in various manners within a range understandable to those skilled in the art based on the content of the present disclosure.

The memory 120 may structure, organize and manage data, combinations of data, program codes executable by the processor 110, ant the like which are required for the processor 110 to perform operations. For example, the memory 120 may store the medical data received via the network unit 130 to be described later. The memory 120 may store program codes that operate a machine learning model to receive medical data and perform training, program codes that operate a machine learning model to receive medical data and perform inference according to the intended use of the computing device 100, and processed data that is generated as the program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network(LAN), a wideband code division multiple access(WCDMA), a long term evolution(LTE), a wireless broadband Internet(WiBro), a 5th generation mobile communication(5G), a ultra wide-band wireless communication, a ZigBee network, a radio frequency(RF) communication, a wireless LAN, a wireless fidelity, a near field communication (NFC), and a Bluetooth network. Since the above-described communication systems are only examples, the wired/wireless communication system for the data transmission and reception of the network unit 130 may be applied in various manners other than the above-described examples.

The network unit 130 may receive data required for the processor 110 to perform operations through wired/wireless communication with any system, any client, or the like. Furthermore, the network unit 130 may transmit the data generated by the operation of the processor 110 through wired/wireless communication with any system, any client, or the like. For example, the network unit 130 may receive medical data through communication with a database within a hospital environment, a cloud server performing tasks such as standardizing medical data, a client such as a smart watch, and a medical computing device. The network unit 130 may transmit output data of a machine learning model, intermediate data and processed data obtained in the operation process of the processor 110, etc. through communication with the above-described database, server, client, or computing device.

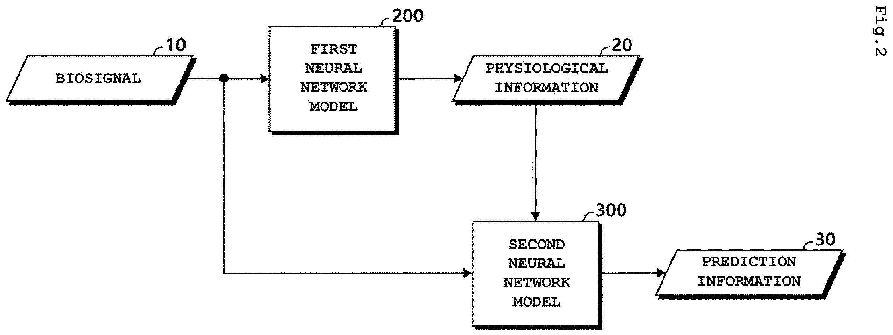

FIG. 2 is a block diagram illustrating an operation process of a neural network model according to one embodiment of the present disclosure.

Referring to FIGS. 1 and 2, the processor 110 according to one embodiment of the present disclosure may train the first neural network model 200 to generate the physiological information 20 on the basis of the biosignal 10. For example, the processor 110, by inputting an electrocardiogram signal to the first neural network model, may generate the physiological information such as age, sex, weight, height, and underlying disease of a subject whose electrocardiogram signal is measured. At this time, the first neural network model 200 may comprise a plurality of sub-models separately constructed for respective types of the physiological information. That is, the first neural network model 200 may comprise sub-models prepared for respective types of the physiological information to be used in predicting disease occurrence, such as a sub-model for generating age information, a sub-model for generating sex information, and a sub-model for generating weight information. When physiological information is generated by the sub-models comprised in the first neural network model 200, the processor 110 may calculate, by using a loss function, an error between the physiological information generated by the sub-models and the actual information of the subject whose electrocardiogram signal is measured. The processor 110 may adjust neural network parameters constituting the first neural network model 200 on the basis of the calculated error. The processor 110 may train the first neural network model 200 by repeating this process.

The processor 110 may train the second neural network model 300, which operates in association with the first neural network model 200, so that the second neural network model 300 predicts disease occurrence in a subject whose biosignal is measured. For example, the processor 110 may input, to the second neural network model 300, the electrocardiogram signal input to the first neural network model 200 together with the physiological information generated by the first neural network model 200, thereby generating prediction information regarding disease occurrence in a subject whose electrocardiogram signal is measured. At this time, the second neural network model 300 may comprise a plurality of sub-models separately constructed for respective types of diseases. That is, the second neural network model 300 may comprise sub-models prepared for respective types of diseases to be predicted, such as a sub-model for predicting occurrence of myocardial infarction, a sub-model for predicting arrhythmia, and a sub-model for predicting stroke. When prediction information is generated by the sub-models comprised in the second neural network model 300, the processor 110 may calculate, by using a loss function, an error between the prediction information generated by the sub-models and the actual information regarding disease occurrence in a subject whose electrocardiogram signal is measured. The processor 110 may adjust neural network parameters constituting the second neural network model 300 on the basis of the calculated error. The processor 110 may train the second neural network model 300 by repeating this process.

Meanwhile, the first neural network model 200 or the second neural network model 300 may be a transformer-based model. However, the type of the first neural network model 200 or the second neural network model 300 is not limited to the examples described above. Furthermore, the training of the first neural network model 200 or the second neural network model 300 is not limited to supervised learning as in the examples described above, and may also be performed by a method such as reinforcement learning which those skilled in the art can apply as an extension of the content of the present disclosure.

When a biosignal of a subject to be predicted is obtained, the processor 110 may generate the physiological information 20 of the subject on the basis of the biosignal 10 using the pre-trained first neural network model 200. At this time, the physiological information 20 may comprise information relating to biological function of the subject, such as age, sex, height, weight, blood pressure, and underlying disease. The processor 110 may generate the prediction information 30 regarding disease occurrence in the subject on the basis of the biosignal 10 and the physiological information 20 generated by the first neural network model 200, using the pre-trained second neural network model 300. The prediction information 30 may comprise information on a probability that a target disease has occurred in the subject. In other words, the processor 110 may generate, through the first neural network model 200, the physiological information matched to the biosignal as additional information, and may utilize the same for predicting disease occurrence through the second neural network model 300. The processor 110 may thereby perform prediction with higher accuracy than in a case of predicting disease occurrence using only biosignal. Furthermore, the processor 110 may perform prediction by generating the physiological information matched to biosignal even in a situation where physiological information other than the biosignal is not obtained.

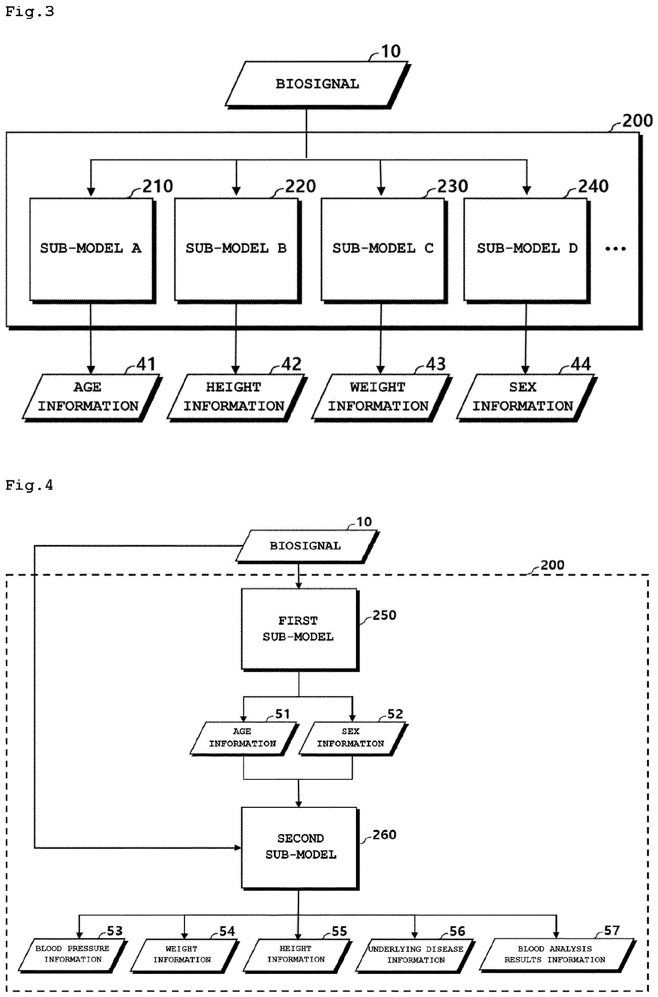

FIG. 3 is a block diagram illustrating a structure of a first neural network model according to one embodiment of the present disclosure.

Referring to FIG. 3, the first neural network model 200 according to one embodiment of the present disclosure may comprise a plurality of sub-models separately constructed for respective types of the physiological information to be utilized for predicting disease occurrence. For example, the first neural network model 200 may comprise: sub-model A 210 that receives the biosignal 10 and generates age information 41; sub-model B 220 that receives the biosignal 10 and generates height information 42; sub-model C 230 that receives the biosignal 10 and generates weight information 43; and sub-model D 240 that receives the biosignal 10 and generates sex information 44. The plurality of sub-models 210, 220, 230, and 240 may be trained individually, and may be operated individually under user control. If the user desires that height information 42 not be utilized for prediction, the user may, through user input, cause sub-model B 220 comprised in the first neural network model 200 not to operate, and cause only the remaining sub-models to operate so that only the required information is generated. As such, by comprising a plurality of sub-models matched to respective types of the physiological information, the first neural network model 200 may generate all additional information that can be utilized for prediction based on the biosignal, or may selectively generate only information that the user needs.

FIG. 4 is a block diagram illustrating a structure of a first neural network model according to an alternative embodiment of the present disclosure.

Referring to FIG. 4, the first neural network model 200 according to an alternative embodiment of the present disclosure may comprise: the first sub-model 250 configured to receive the biosignal 10 and generate first additional information included in the physiological information; and the second sub-model 260 configured to receive the biosignal 10 together with the first additional information generated by the first sub-model 250 and generate second additional information included in the physiological information. For example, the first sub-model 250 may receive the biosignal 10 and generate first additional information comprising at least one of the age information 51 or the sex information 52. The Age information 51 and the sex information 52 may be generated individually by a plurality of subordinate models comprised in the first sub-model 250.

The second sub-model 260 may receive, together with the biosignal 10, at least one of the age information 51 or the sex information 52 comprised in the first additional information, and may generate second additional information comprising at least one of the blood pressure information 53, the weight information 54, the height information 55, the underlying disease information 56, or the blood analysis results information 57. At this time, the blood analysis results information 57 may be numerical information regarding substances that can be determined by blood analysis, such as blood glucose level, anemia index, and electrolyte concentration. For example, the second sub-model 260 may generate the height information 56 on the basis of the biosignal 10, the age information 51, and the sex information 52. The second sub-model 260 may generate the weight information 54 on the basis of the biosignal 10, the age information 51, the sex information 52, the height information 55, and the blood pressure information 53. The second sub-model 260 may generate the underlying disease information 56 regarding whether an underlying disease such as asthma is present, on the basis of the biosignal 10, the age information 51, and the sex information 52. The second neural network model 300 may receive the biosignal 10, the first additional information generated by the first sub-model 250, and the second additional information generated by the second sub-model 260, and may calculate a probability of disease occurrence in a subject to be predicted.

Through this structure, the first neural network model 200 may stably generate even physiological information that is relatively difficult to predict from the biosignal 10 alone, such as weight. That is, by employing a hierarchical structure comprising the first sub-model 250 and the second sub-model 260, the first neural network model 200 may distinguish physiological information that is relatively easy to predict from physiological information that is relatively difficult to predict from the biosignal 10, and may complementarily generate the relatively difficult-to-predict physiological information using the relatively easy-to-predict physiological information together with the biosignal 10. Accordingly, this structure of the first neural network model 200 may also ensure performance of final disease prediction executed by the second neural network model 300.

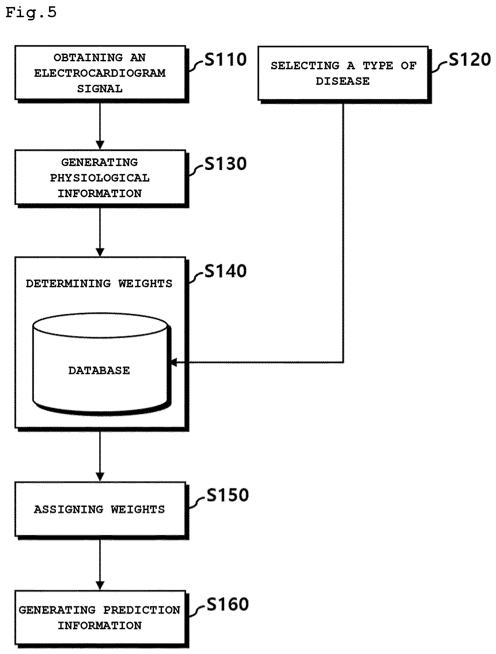

FIG. 5 is a flowchart illustrating a process of generating prediction information according to one embodiment of the present disclosure.

Referring to FIGS. 1 and 5, the computing device 100 according to one embodiment of the present disclosure may obtain an electrocardiogram signal through communication with an electrocardiogram measuring device or a medical server S110. The computing device 100 may obtain a selection input regarding a type of disease that a user desires to predict through communication with an electrocardiogram measuring device or a medical server S120. For example, when a medical institution measures an electrocardiogram signal using a 12-lead electrocardiogram measuring device, a clinician may transmit the electrocardiogram signal to the computing device 100 through an interface capable of interworking with the computing device 100. The clinician may also transmit, through the interface, a selection input of a disease to be predicted together with the electrocardiogram signal to the computing device 100.

The computing device 100 may generate the physiological information on the basis of an electrocardiogram signal through the pre-trained first neural network model S130. At this time, the physiological information is information related to a subject to be predicted whose electrocardiogram signal is measured, and may include age, weight, sex, blood pressure, blood analyte levels, presence or absence of underlying disease, and the like. The computing device 100 may determine, according to the type of disease determined by a user input, weights to be assigned to a plurality of additional information included in the physiological information generated by the first neural network model S140. The computing device 100 may assign the weights determined through the step S140 to the plurality of additional information included in the physiological information S150. The computing device 100 may, by using the pre-trained second neural network model, generate the prediction information regarding occurrence of the disease determined by a user input on the basis of the electrocardiogram signal and the plurality of additional information to which the weights determined through the step S140 are assigned S160.

For example, when a clinician selects myocardial infarction as a disease to be predicted through the interface, the computing device 100 may select, from the weight database constructed based on analysis results on correlations between additional information included in the physiological information and specific diseases by clinical experts, weights that are matched with myocardial infarction. That is, the weights to be assigned to the plurality of additional information may vary depending on the type of disease. Furthermore, the computing device 100 may dynamically select, from the weight database constructed based on analysis results by clinical experts, weights to be assigned to the plurality of additional information included in the physiological information according to a user input. The computing device 100 may assign the weights selected from the weight database to the physiological information generated by the first neural network model. The computing device 100 may then input the electrocardiogram signal and the weighted physiological information to the second neural network model. At this time, since the second neural network model comprises a plurality of sub-models separately constructed for respective types of diseases, the computing device 100 may activate, among the plurality of sub-models included in the second neural network model, a model for predicting myocardial infarction and calculate a probability of occurrence of myocardial infarction. That is, when the type of disease is determined by a user input, a sub-model of the second neural network model corresponding to the disease determined by the user input is activated to predict disease occurrence.

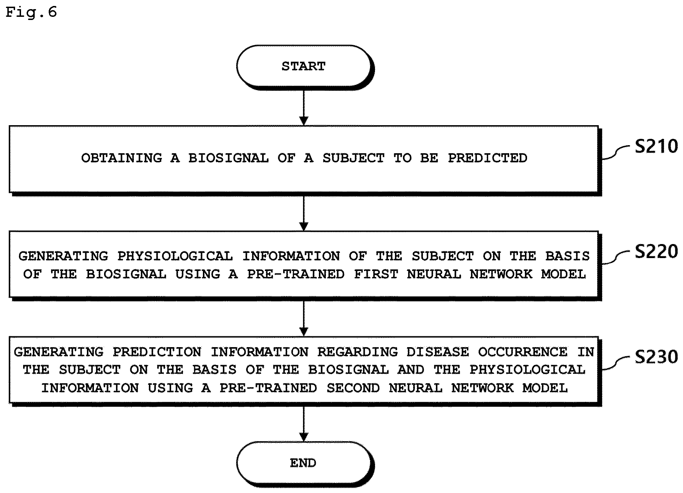

FIG. 6 is a flowchart illustrating an artificial intelligence-based disease prediction method according to one embodiment of the present disclosure.

Referring to FIGS. 1 and 6, the computing device 100 according to one embodiment of the present disclosure may obtain the biosignal of a subject to be predicted S210.

The computing device 100 may generate the physiological information of the subject on the basis of the biosignal obtained through the step S210, using the pre-trained first neural network model S220. For example, the first neural network model may be a model that individually predicts, as additional information for disease prediction, the physiological information such as height, age, and weight utilizing the biosignal as input data. Particularly, the first neural network model may comprise a sub-model for predicting age, a sub-model for predicting sex, a sub-model for predicting weight, a sub-model for predicting blood pressure, a sub-model for predicting diabetes as an underlying disease, and the like. Accordingly, the first neural network model may receive the biosignal and individually generate physiological information such as age, sex, weight, blood pressure, and presence of diabetes through the plurality of sub-models.

The computing device 100 may generate the prediction information regarding disease occurrence in a subject to be predicted on the basis of the biosignal obtained through the step S210 and the physiological information generated through the step S220, using the pre-trained second neural network model S230. For example, the second neural network model may calculate a probability of occurrence of a specific disease by receiving the biosignal together with the physiological information generated by the first neural network model. When the second neural network model comprises a plurality of sub-models so as to predict a plurality of diseases, a sub-model of the second neural network model corresponding to a disease specified by a user may operate to calculate, on the basis of the biosignal and the physiological information, a probability of occurrence of the disease specified by the user.

The various embodiments of the present disclosure described above may be combined with additional embodiments and may be modified within a range understandable to those skilled in the art based on the foregoing detailed description. The embodiments of the present disclosure are to be understood as illustrative in all respects and not limiting. For example, individual components described as being implemented in a unitary form may be implemented in a distributed manner, and likewise, components described as being implemented in a distributed manner may be implemented in a combined form. Therefore, all changes or modifications derived from the meaning, scope, and equivalents of the claims of the present disclosure are to be construed as being included within the scope of the present disclosure.

## Claims

1. A method of predicting a disease based on artificial intelligence, the method to be performed by a computing device including at least one processor, the method comprising:
obtaining a biosignal of a subject to be predicted;
generating physiological information of the subject on the basis of the obtained biosignal using a pre-trained first neural network model; and
generating prediction information regarding disease occurrence in the subject on the basis of the obtained biosignal and the generated physiological information using a pre-trained second neural network model.

2. The method of claim 1, wherein
the first neural network model comprises
a plurality of sub-models separately constructed for respective types of the physiological information.

3. The method of claim 1, wherein
the first neural network model comprises:
a first sub-model configured to receive the obtained biosignal and generate first additional information included in the physiological information; and
a second sub-model configured to receive the obtained biosignal together with the generated first additional information, and generate second additional information included in the physiological information.

4. The method of claim 3, wherein
the first additional information
comprises at least one of age or sex of the subject, and
the second additional information
comprises at least one of blood pressure, blood analysis results, weight, height, or underlying disease of the subject.

5. The method of claim 3, wherein
the second neural network model
receives the obtained biosignal, the generated first additional information, and the generated second additional information, and calculates a probability of disease occurrence in the subject.

6. The method of claim 1, wherein
generating prediction information regarding disease occurrence in the subject on the basis of the obtained biosignal and the generated physiological information using the pre-trained second neural network model comprises:
determining weights to be assigned to a plurality of additional information included in the generated physiological information according to a type of disease determined by a user input; and
generating prediction information regarding occurrence of the disease determined by the user input, on the basis of the obtained biosignal and the plurality of additional information to which the determined weights are assigned, using the second neural network model.

7. The method of claim 6, wherein
the weights to be assigned to the plurality of additional information
vary depending on the type of disease, and
are dynamically selected from a weight database constructed based on analysis results of clinical experts according to the user input.

8. The method of claim 6, wherein
the second neural network model
comprises a plurality of sub-models separately constructed for respective types of diseases, and
wherein when the type of disease is determined by the user input, a sub-model of the second neural network model corresponding to the determined type of disease is activated.

9. A computer program stored in a computer-readable storage medium, the computer program, when executed by one or more processors, performing operations for predicting a disease based on artificial intelligence,
wherein the operations comprise:
obtaining a biosignal of a subject to be predicted;
generating physiological information of the subject on the basis of the obtained biosignal using a pre-trained first neural network model; and
generating prediction information regarding disease occurrence in the subject on the basis of the obtained biosignal and the generated physiological information using a pre-trained second neural network model.

10. A computing device for predicting a disease based on artificial intelligence, the computing device comprising:
a processor including at least one core;
a memory including program codes executable by the processor; and
a network unit configured to obtain a biosignal of a subject to be predicted,
wherein the processor is configured to:
generate physiological information of the subject on the basis of the obtained biosignal using a pre-trained first neural network model; and
generate prediction information regarding disease occurrence in the subject on the basis of the obtained biosignal and the generated physiological information using a pre-trained second neural network model.
